# EUROPEAN PATENT APPLICATION

(11) **EP 4 321 861 A1**
(43) Date of publication of application: **14.02.2024**
(21) Application number: 22784731.6
(22) Date of filing: 08.04.2022
(51) Int. Cl.: G01N 27/62, G01N 30/72, G01N 33/50, H01J 49/00, H01J 49/26

(54) **ANALYSIS METHOD AND PRETREATMENT APPARATUS**

(30) Priority: 09.04.2021 JP 2021066338
(71) Applicant: Shimadzu Corporation, Nakagyo-ku, Kyoto-shi, Kyoto 604-8511 (JP); Kyushu University, National University Corporation, Nishi-ku Fukuoka-shi Fukuoka 819-0395 (JP)
(72) Inventor: KAWAKAMI, Daisuke, Kyoto-shi, Kyoto 604-8511 (JP); SETOYAMA, Daiki, Fukuoka-shi, Fukuoka 819-0395 (JP); KANG, Dongchon, Fukuoka-shi, Fukuoka 819-0395 (JP); UEYANAGI, Yasushi, Fukuoka-shi, Fukuoka 819-0395 (JP)
(74) Representative: Müller-Boré & Partner Patentanwälte PartG mbB
(86) International application number: PCT/JP2022/017358
(87) International publication number: WO 2022/215749

(57) **Abstract**

In an analysis system (1000), a pretreatment apparatus (400) causes a sample related to a metabolic disorder (a sample based on a metabolite of a subject) with a reagent. The reagent contains 3-nitrophenylhydrazine. By causing the sample to react with the reagent, an organic acid contained in the sample is derivatized. Pretreatment apparatus (400) transports the sample containing the organic acid derivatized to an LC mass spectrometer (100). LC mass spectrometer (100) analyzes the sample that has been transported.

## Description

### TECHNICAL FIELD

The present invention relates to analysis of samples relating to a metabolic disorder.

### BACKGROUND ART

One of literatures that disclose inspection of the metabolic disorder is current status of expansion screening for a congenital metabolic disorder using tandem masses, Tomiko Kuhara, Japanese Journal of Hygiene, THE JAPANESE SOCIETY FOR HYGIENE, 2014, 69, pp. 60-74. The literature discloses a simple inspection (primary screening) and a confirmation inspection (secondary screening) that is more precise than the simple inspection and is performed based on results of the simple inspection. The literature also discloses a method using gas chromatography-mass spectrometry (GC/MS) as one of analysis methods of a sample to be used in the confirmation inspection. The literature further discloses that as for an inspection method using the GC/MS, information and data are overwhelmingly accumulated more as compared with an inspection method using other analysis methods such as liquid chromatography-mass spectrometry (LC/MS). Then, conventionally, the GC/MS has been adopted as an analysis method for confirmation inspection of the metabolic disorder.

### CITATION LIST

### NON PATENT LITERATURE

NPL 1: Present Status of Expanded Newborn Screening Project for Inborn Errors of Metabolism by Tandem Mass Spectrometry, Tomiko Kuhara, Japanese Journal of Hygiene, THE JAPANESE SOCIETY FOR HYGIENE, 2014, 69, pp. 60-74.

### SUMMARY OF INVENTION

### TECHNICAL PROBLEM

Precise inspection results for the metabolic disorder, may be immediately required, such as in a case where a patient's condition changes rapidly. However, analysis by the GC/MS requires a long time for sample pretreatment (extraction of organic acids with an organic solvent, drying and solidification of an organic layer, and the like). Therefore, there has been a demand for a technique for enabling the sample to be analyzed in a short time, thereby enabling the confirmation inspection of the metabolic disorder to be performed in a short time.

The present invention has been made in view of such circumstances, and an object thereof is to provide a technique for performing the precise inspection for the metabolic disorder in a short time.

### SOLUTION TO PROBLEM

An analysis method according to an aspect of the present disclosure is an analysis method for a sample related to the metabolic disorder, the method including: derivatizing a sample, which is based on a metabolite of a subject, by reacting with a reagent containing 3-nitrophenylhydrazine; introducing the derivatized sample into a liquid chromatograph mass spectrometry apparatus; and analyzing, by the liquid chromatograph mass spectrometry apparatus, the sample derivatized.

An analysis method according to another aspect of the present disclosure is an analysis method for a sample related to a metabolic disorder, the method including: receiving an input of an analysis type; analyzing a sample, which is based on a metabolite of a subject, according to a first flow by using a liquid chromatograph mass spectrometry apparatus in a case where the input is a first type of input; and analyzing the sample, which is based on the metabolite of the subject, according to a second flow by using the liquid chromatograph mass spectrometry apparatus in a case where the input is a second type of input. The first flow includes: derivatizing a reagent by reacting with a reagent containing 3-nitrophenylhydrazine; introducing the derivatized sample into the liquid chromatograph mass spectrometry apparatus; and analyzing, by the liquid chromatograph mass spectrometry apparatus, the sample derivatized, and the second flow includes: introducing the sample without derivatization into the liquid chromatograph mass spectrometry apparatus; and analyzing the sample without derivatization by the liquid chromatograph mass spectrometry apparatus.

An analysis method according to still another aspect of the present disclosure is an analysis method for a sample related to a metabolic disorder, the method including: adding alcohol to a sample based on a metabolite of a subject; filtering the alcohol-added sample; derivatizing, by reacting with a reagent, the filtered sample; introducing the derivatized sample into a liquid chromatograph mass spectrometry apparatus; and analyzing, by the liquid chromatograph mass spectrometry apparatus, the sample derivatized.

A pretreatment apparatus according to an aspect of the present disclosure is a pretreatment apparatus performing pretreatment on a sample related to a metabolic disorder, the apparatus including: a derivatizing unit that derivatizes a sample, which is based on a metabolite of a subject, by reacting with a reagent containing 3-nitrophenylhydrazine; and a transporting unit that introduces the derivatized sample into a liquid chromatograph mass spectrometry apparatus.

A pretreatment apparatus according to another aspect of the present disclosure is a pretreatment apparatus performing pretreatment on a sample related to a metabolic disorder, the apparatus including: an input unit that receives an input of an analysis type; a derivatizing unit that derivatizes a sample by reacting with a reagent containing 3-nitrophenylhydrazine; and a transporting unit that introduces the sample into a liquid chromatograph mass spectrometry apparatus. In a case where the input is a first type of input, the derivatizing unit derivatizes a sample, which is based on a metabolite of a subject, by reacting with the reagent, and the transporting unit introduces the derivatized sample by the derivatizing unit into the liquid chromatograph mass spectrometry apparatus. In a case where the input is a second type of input, the transporting unit introduces the sample without derivatization by the derivatizing unit into the liquid chromatograph mass spectrometry apparatus.

A pretreatment apparatus according to still another aspect of the present disclosure is a pretreatment apparatus performing pretreatment on a sample related to a metabolic disorder, the apparatus including: an adding unit that adds alcohol to a sample based on a metabolite of a subject; a filtering unit that filters the alcohol-added sample; a derivatizing unit that derivatizes, by reacting with a reagent, the filtered sample; and a transporting unit that introduces the delivatized sample into a liquid chromatograph mass spectrometry apparatus.

### ADVANTAGEOUS EFFECTS OF INVENTION

According to an aspect of the present disclosure, in the analysis method described in Item 1, an organic acid contained in the sample is derivatized by reacting with the reagent containing the 3-nitrophenylhydrazine, and thus derivatized in a short time.

According to another aspect of the present disclosure, the liquid chromatograph mass spectrometry apparatus is used in analysis for the confirmation inspection and analysis for the simple inspection. Further, in the analysis for the confirmation inspection, the organic acid contained in the sample is derivatized by reacting with the reagent containing the 3-nitrophenylhydrazine, and thus derivatized in a short time.

According to still another aspect of the present disclosure, drying and solidification of the organic solvent is not required in the derivatization, and thus the organic acid contained in the sample is derivatized in a short time.

### BRIEF DESCRIPTION OF DRAWINGS

Fig. 1 is a schematic configuration diagram of an analysis system 1000 according to one embodiment of the present disclosure.
Fig. 2 is a diagram illustrating 25 types of diseases.
Fig. 3 is a diagram illustrating specific examples of organic acids to be measured in analysis system 1000 for determination of presence or absence of a disease and the like.
Fig. 4 is an example of a chemical reaction formula illustrating a derivatization reaction in a derivatizing unit 406.
Fig. 5 is a flowchart illustrating an example of an analysis method for a sample related to a metabolic disorder by analysis system 1000.
Fig. 6 is a diagram illustrating an example of a recovery rate of each of the 23 types of organic acids.
Fig. 7 is a flowchart illustrating a variation of the analysis method illustrated in Fig. 5.

### DESCRIPTION OF EMBODIMENTS

Hereinafter, embodiments of the present disclosure will be described in detail with reference to the drawings. Note that, in the drawings, the same or corresponding portions are denoted by the same reference numerals, and descriptions of the portions will not be repeated. Although numerical values such as amounts, temperatures, and concentrations of the reagent and the like are disclosed in the embodiments of the present disclosure, the numerical values include not only values themselves disclosed, but also a range in which effects equivalent to effects exhibited by approximate values of the values disclosed or the values disclosed are exhibited.

### [Configuration of analysis system]

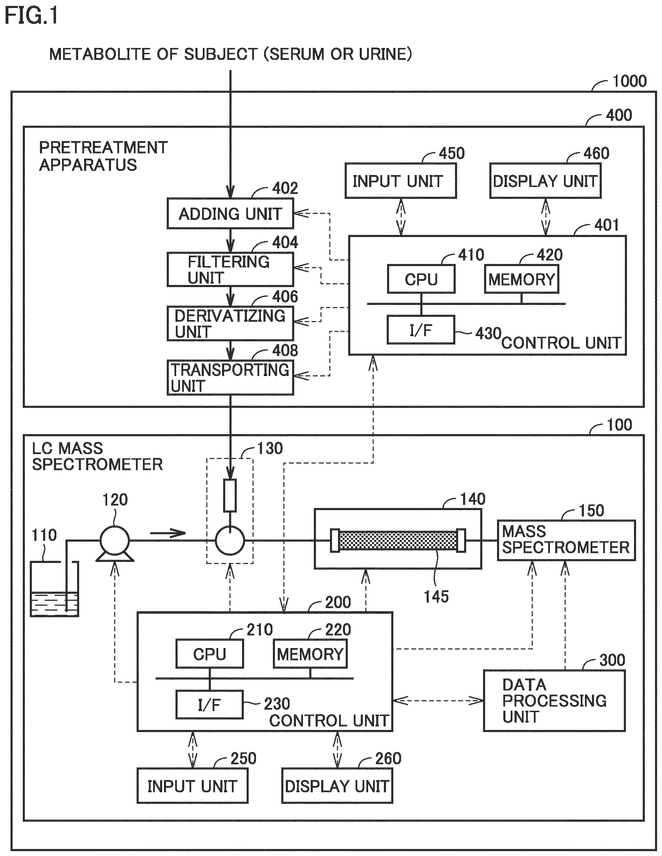

Fig. 1 is the schematic configuration diagram of analysis system 1000 according to one embodiment of the present disclosure. Referring to Fig. 1, analysis system 1000 includes a liquid chromatograph mass spectrometer 100 (hereinafter, referred to as "LC mass spectrometer 100") and a pretreatment apparatus 400.

### [Configuration of LC mass spectrometer]

Next, a configuration of LC mass spectrometer 100 will be described with reference to Fig. 1. LC mass spectrometer 100 includes a solvent container 110 that stores a mobile phase, a liquid feeding pump 120, an injector 130, a column oven 140, a mass spectrometer 150, a control unit 200, and a data processing unit 300.

Control unit 200 includes a central processing unit (CPU) 210, a memory 220, and an interface (I/F) 230. Control unit 200 controls operations of elements (for example, liquid feeding pump 120, mass spectrometer 150, data processing unit 300, and a display unit 260) in LC mass spectrometer 100. Control unit 200 is connected to an input unit 250 and display unit 260, all of which are user interfaces via I/F 230. The connection may be wired or wireless.

Input unit 250 is, for example, a keyboard and/or a pointing device (such as a mouse), and receives an input from a user (for example, a person in charge of analysis). Display unit 260 includes, for example, a liquid crystal panel, and displays the information to the user. In a case where a touch panel is used as the user interface, input unit 250 and display unit 260 are integrally formed.

Liquid feeding pump 120 sucks a mobile phase of a liquid from solvent container 110 and supplies the liquid toward a column 145 at a constant flow rate. Injector 130 injects a sample to be analyzed into the mobile phase supplied by liquid feeding pump 120.

Injector 130 injects the sample to be analyzed into the mobile phase at a timing based on instructions from control unit 200. The sample injected is supplied to column 145 along with a flow of the mobile phase fed by liquid feeding pump 120. Injector 130 includes, for example, an autosampler. The sample may be introduced from pretreatment apparatus 400 to injector 130.

Column oven 140 accommodates column 145. Control unit 200 adjusts an ambient temperature of column 145 to a predetermined temperature in column oven 140.

The sample injected into injector 130 is introduced into column 145 together with the liquid of the mobile phase. Column 145 separates various components in the sample.

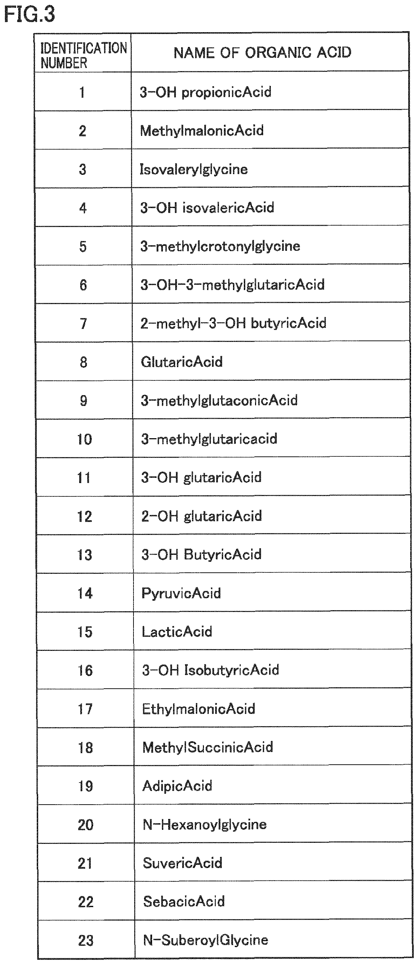

Mass spectrometer 150 extracts a target component to be detected as an ion, and detects a concentration (and/or an amount) of the target component by measuring the ion. Mass spectrometer 150 outputs data indicating a detected value to data processing unit 300. The target component is, for example, one of the 23 types of organic acids illustrated in Fig. 3 to be described later.

Mass spectrometer 150 may identify the concentration of the target component according to an internal standard method. The internal standard method is a method for determining the concentration of the target component based on a relationship between a peak area ratio and a concentration ratio of each of the target component and an internal standard substance.

Data processing unit 300 generates a chromatogram by processing the data indicating the detected value. Data processing unit 300 is embodied, for example, by operating, on a computer, dedicated software installed in the computer. Data processing unit 300 stores data output from mass spectrometer 150 as chromatogram data based on an instruction from control unit 200, and creates a chromatogram based on the chromatogram data obtained. The chromatogram created is stored in memory 220 inside control unit 200. Control unit 200 displays the chromatogram on display unit 260 in response to a request from the user. Control unit 200 also transmits the chromatogram to pretreatment apparatus 400 in response to an instruction from pretreatment apparatus 400.

### [Metabolic disorder]

Analysis system 1000 outputs data for determining presence or absence of the metabolic disorder of the subject by analyzing the metabolite of the subject. The metabolite of the subject is, for example, a liquid, typically serum, plasma or urine. The metabolite of the subject may be whole blood. Analysis system 1000 can further output data for determining a type of the metabolic disorder. Hereinafter, the metabolic disorder may be the congenital metabolic disorder, and is also referred to as "disease".

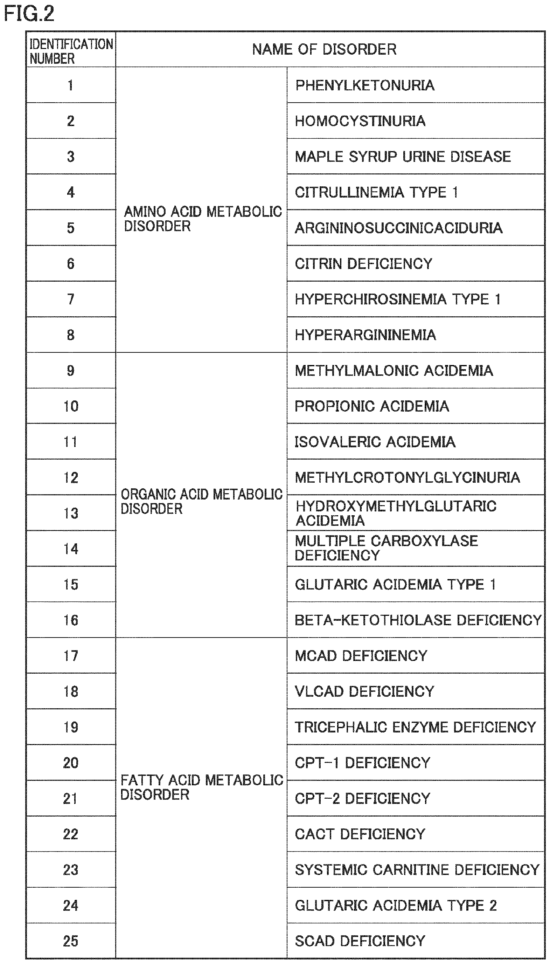

Fig. 2 is the diagram illustrating the 25 types of diseases. As illustrated in an example of Fig. 2, the diseases are roughly classified into an amino acid metabolic disorder, an organic acid metabolic disorder, and a fatty acid metabolic disorder. In the example of Fig. 2, the amino acid metabolic disorder includes phenylketosis (identification number 1). The organic acid metabolic disorder includes methylmalonic acidemia (identification number 9). The fatty acid metabolic disorder includes tricephalic enzyme deficiency (identification number 19).

Fig. 3 is the diagram illustrating the specific examples of the organic acids to be measured in analysis system 1000 for the determination of the presence or the absence of the disease and the like. In Fig. 3, 23 types of compounds are illustrated. Note that, in Fig. 3, "hydroxy" indicating a hydroxy group is represented by "OH".

In an example of Fig. 3, examples of the organic acids contained in the metabolite include 3-hydroxypropionic acid (identification number 1), methylmalonic acid (identification number 2), isovalerylglycine (identification number 3), 3-isovaleric acid (identification number 4), 3-methylcrotonylglycine (identification number 5), 3-hydroxy-3-methylglutaric acid (identification number 6), 2-methyl-3-hydroxybutyl acid (identification number 7), glutaric acid (identification number 8), 3-methylglutaric acid (identification number 9), 3-methylglutaric acid (identification number 10), 3-hydroxyglutaric acid (identification number 11), 2-hydroxyglutaric acid (identification number 12), 3-hydroxybutyl acid (identification number 13), pyruvic acid (identification number 14), lactic acid (identification number 15), 3-hydroxyisobutyric acid (identification number 16), ethylmalonic acid (identification number 17), methylsuccinic acid (identification number 18), adipic acid (identification number 19), N-hexanoylglycine (identification number 20), suberic acid (identification number 21), sebacic acid (identification number 22), and suberoylglycine (identification number 23). The organic acids to be measured (the organic acids for determining the presence or the absence of the disease in the subject) include at least one selected from the group consisting of identification numbers 1 to 23.

In analysis system 1000, mass spectrometer 150 derives detected values (for example, concentrations of the organic acids in the sample) of the organic acids (target components). Analysis system 1000 may output the detected value of each of the organic acids as analysis results. Doctors can determine presence or absence of a disease corresponding to the each of the organic acids and a type of the disease based on whether or not the detected value of the each of the organic acids falls in a predetermined normal range for the each of the organic acids.

For example, in a case of determining that a detected value of the 3-hydroxypropionic acid (identification number 1 in Fig. 3) among the organic acids contained in the metabolite of the subject does not fall in the normal range, the doctors determine that the disease in the subject is propionic acidemia (identification number 10 in Fig. 2).

Further, in a case of determining that a detected value of the methylmalonic acid (identification number 2 in Fig. 3) among the organic acids contained in the metabolite of the subject does not fall in the normal range, the doctors determine that the disease of the subject is methylmalonic acidemia (identification number 9 in Fig. 2).

### [Configuration of pretreatment apparatus]

The configuration of pretreatment apparatus 400 will be described with reference to Fig. 1 again. Pretreatment apparatus 400 includes a control unit 401, an adding unit 402, a filtering unit 404, derivatizing unit 406, and a transporting unit 408.

Control unit 401 includes a CPU 410, a memory 420, and an I/F 430. Control unit 401 controls operations of elements (for example, adding unit 402, filtering unit 404, derivatizing unit 406, transporting unit 408, and a display unit 460) in pretreatment apparatus 400. Control unit 401 is connected to an input unit 450 and display unit 460 via I/F 430. The connection may be wired or wireless. Input unit 450 is, for example, the keyboard and/or the pointing device (such as the mouse), and receives an input from the user. Display unit 460 includes, for example, the liquid crystal panel, and displays the information to the user. In the case where the touch panel is used as the user interface, input unit 450 and display unit 460 are integrally formed.

Control unit 401 integrally controls analysis system 1000. In other words, control unit 401 is connected to control unit 200 of LC mass spectrometer 100 via I/F 430 and I/F 230, and transmits instructions to control unit 200. The connection may be wired or wireless.

Pretreatment apparatus 400 receives the sample based on the metabolite (the serum, the plasma or the urine) of the subject. The sample based on the metabolite may be blood obtained from a dried blood sample, the metabolite itself collected from the subject, or a blood sample specimen.

Adding unit 402 adds a deproteinization solution to the sample. Adding unit 402 includes a mechanism for adding (dispensing) the deproteinization solution to the sample. The solution contains a solvent and an internal standard substance corresponding to an organic acid to be measured. The solvent is, for example, the alcohol. The internal standard substance is, for example, 2-ethylbutyric acid.

The above sample to which the above solution is added is filtered by filtering unit 404 to remove protein from the substance. Filtering unit 404 includes, for example, a mechanism for performing suction filtration by using a Teflon (registered trademark) filter having a thickness of 0.45 µm.

The sample from which the protein is removed is derivatized by derivatizing unit 406. More specifically, derivatizing unit 406 derivatizes a carboxyl group of the organic acid contained in the sample by causing a reagent to react with a reagent in the sample from which the protein is removed. Derivatizing unit 406 includes, for example, a mechanism for dispensing the above reagent into the sample. Ionization efficiency can be improved in mass spectrometer 150 by the derivatization. Further, a molar mass of the organic acid can be increased by the derivatization, and thus analysis accuracy by mass spectrometer 150 can be improved.

Next, the reagent used in the derivatization will be described. The reagent is produced by mixing a first substance with a second substance in the same amount as the first substance.

The first substance is, for example, a substance in which the 3-nitrophenylhydrazine (hereinafter, also referred to as "3-NPH") is dissolved in 75% methanol to make a concentration of the 3-NPH 200 mM.

The second substance is a substance in which 1-ethyl-3-(3-dimethylaminopropyl)carbodiimide (hereinafter, also referred to as "EDC") and pyridine are dissolved in the 75% methanol to make a concentration of the EDC 200 mM and a concentration of pyridine 9%.

The 75% methanol is produced, for example, by mixing the methanol with water. "M" represents a molar concentration (mol/liter). The concentration of the pyridine, "%", represents a weight concentration (weight/volume).

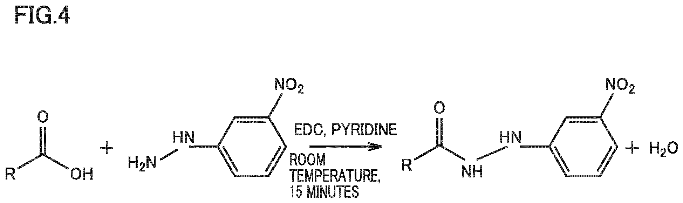

Fig. 4 is the example of the chemical reaction formula illustrating the derivatization reaction in derivatizing unit 406. A first term on a left side of the chemical reaction formula in Fig. 4 represents an organic acid (carboxylic acid) contained in the sample. A second term on the left side represents the 3-NPH. A right side represents the organic acid derivatized. In the present description, in a case where the organic acid contained in the sample is derivatized, the sample in a state of containing the organic acid derivatized is referred to as "sample derivatized".

In the example of Fig. 4, the sample (the organic acid) from which the protein is removed is reacted with the reagent at a room temperature (for example, 25 degrees) for 15 minutes. Accordingly, the carboxyl group of the organic acid is derivatized. In the example of Fig. 4, the EDC and the pyridine function as catalysts.

In the organic acid derivatized (a substance illustrated on the right side of the chemical reaction formula), an N-N bond tends to be easily broken. In other words, pretreatment apparatus 400 produces a substance (the organic acid derivatized) that is easily ionized by derivatizing the carboxyl group. Further, a molecular weight (a molar mass) of the substance derivatized is larger than a molecular weight (a molar mass) of the original organic acid. Therefore, pretreatment apparatus 400 produces a substance (the organic acid derivatized) having a molar mass larger than the molar mass of the original organic acid by derivatizing the carboxyl group.

Refer to Fig. 1 again. Transporting unit 408 transports (a container for) the sample in pretreatment apparatus 400 to injector 130 of LC mass spectrometer 100. The sample in pretreatment apparatus 400 may or may not be derivatized before being transported to injector 130.

### [Analysis method]

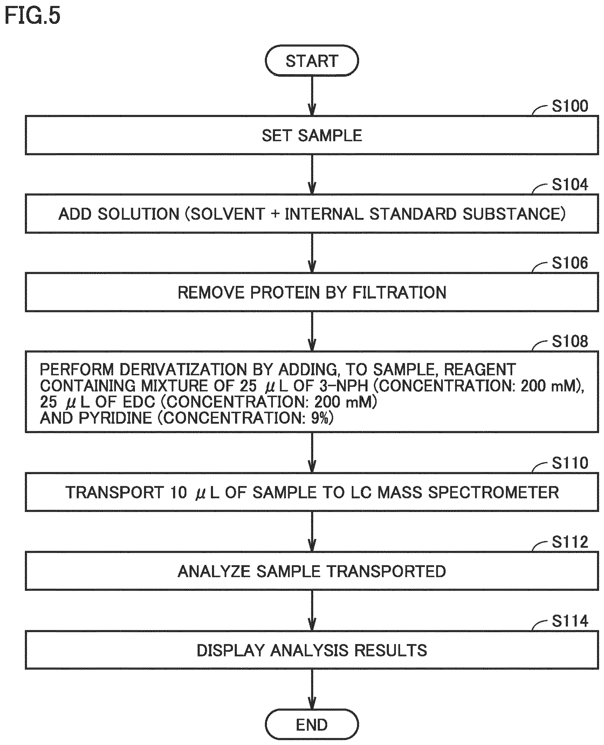

Fig. 5 is the flowchart illustrating the example of the analysis method for the sample related to the metabolic disorder by analysis system 1000. In the example of Fig. 5, 10 µL of the serum of the subject is used as an example of the sample.

First, in step S100, the user sets the sample in pretreatment apparatus 400. The sample may be the metabolite itself of the subject or may be adjusted by the user or pretreatment apparatus 400 from the metabolite of the subject.

Next, in step S104, adding unit 402 adds a solution containing the solvent described above and the internal standard substance to the sample.

Next, in step S106, filtering unit 404 removes the protein from the sample by filtering the sample to which the solution is added in step S104.

Next, in step S108, derivatizing unit 406 derivatizes the sample from which the protein is removed.

In the derivatization, derivatizing unit 406 adds the reagent to the sample from which the protein is removed in step S106. The reagent contains 25 µL of the first substance and 25 µL of the second substance. As described above, the first substance may be the substance containing the 3-NPH at the concentration of 200 mM. The second substance may be the substance containing the EDC at the concentration of 200 mM and the pyridine at the concentration of 9%.

Derivatizing unit 406 achieves reaction conditions for the derivatization after adding the above reagent to the above sample. The reaction conditions are, for example, maintaining the room temperature (for example, 25°C) for 15 minutes while stirring. Derivatizing unit 406 may include a mechanism for swinging a sample container for the stirring and a mechanism for adjusting a temperature of a region where the sample container is placed.

Next, in step S110, transporting unit 408 transports 10 µL of the sample derivatized to injector 130 of LC mass spectrometer 100.

Next, in step S112, control unit 401 outputs a sample analysis instruction to LC mass spectrometer 100. In response to the instruction, control unit 200 of LC mass spectrometer 100 introduces the sample into mass spectrometer 150 via column 145, and causes mass spectrometer 150 to output the analysis results of the sample. Control unit 200 transmits the analysis results to control unit 401 of pretreatment apparatus 400.

Next, in step S114, control unit 401 displays the analysis results on display unit 460. Control unit 401 may highlight the detected values that do not fall in the predetermined normal range for the each of the organic acids among the detected values of two or more of corresponding organic acids included in the analysis results. Further, control unit 401 may display, on display unit 460 for reference, a name (any of names of the 25 disease illustrated in Fig. 2) of the metabolic disorder associated in advance as to the organic acid with the detected value not falling within the normal range.

### [Summary]

In the embodiment described above, the LC mass spectrometer analyzes the sample based on the metabolite of the subject. According to the embodiment described above, the analysis can be easily performed as compared with analysis using a conventional gas chromatograph mass spectrometer (hereinafter, referred to as "GC mass spectrometer").

More specifically, in the analysis using the GC mass spectrometer, since a substance to be measured is a gas, vaporization of the metabolite is required. Note that, the vaporization of the metabolite by heating is not preferable because a substance for determining the presence or the absence of the metabolic disorder may be decomposed by heat. Therefore, conventionally, in analysis for determination of the methylmalonic acidemia, a process of extracting the organic acid (the methylmalonic acid) by using the organic solvent, a process of drying and solidifying the organic layer extracted, and a process of derivatizing a substance dried and solidified, have been performed as the pretreatment. However, there is a problem that these processes take a large amount of time (for example, 5 hours). Even when a patient who desires to determine whether or not he/she has the metabolic disorder takes an examination at a medical facility, since it takes the large amount of time, the determination may not be completed while the patient is staying in the medical facility. Further, in order to perform the above processes for the pretreatment, the person in charge of analysis is required to have a lot of experience, and it may be difficult for an inexperienced person in charge of analysis to perform these processes.

On the other hand, in the present embodiment, the processes described above are not required. Therefore, according to the present embodiment, the analysis can be easily performed.

In general, the substance (the organic acid) for determining the presence or the absence of the metabolic disorder includes the carboxyl group, the substance being contained in the sample based on the metabolite of the subject. In the embodiment described above, the derivatization of the organic acids is performed by using the reagent containing a component that derivatize the carboxyl group by reacting with the carboxyl group contained in the sample based on the metabolite of the subject, as described above. Accordingly, the embodiment described above can derivatize the organic acids without requiring a long-time pretreatment (including the process of extracting the organic acids by using the organic solvent) conventionally required. Further, the derivatization is performed by pretreatment apparatus 400.

Also in this sense, in the embodiment describe above, by using pretreatment apparatus 400 for the pretreatment, the pretreatment can be easily performed even in a case where the person in charge of analysis is an inexperienced person.

Further, a period of time required for the pretreatment using the conventional GC mass spectrometer is about 5 hours, whereas a period of time required for the pretreatment of the embodiment described above is about 30 minutes. Therefore, it is possible to shorten a period of time required for the pretreatment to determine the metabolic disorder as compared with the related art.

Further, pretreatment apparatus 400 of the embodiment described above can derivatize the carboxyl group in a short time by using the reagent containing the 3-NPH as described as step S 108 in Fig. 5. Accordingly, under the reaction conditions for the derivatization, a reaction period of time of about 15 minutes is considered sufficient.

Moreover, since the above reagent contains the EDC and the pyridine, pretreatment apparatus 400 of the embodiment described above can derivatize the carboxyl group in a shorter time.

Moreover, pretreatment apparatus 400 of the embodiment described above can derivatize the carboxyl group in a short time by using the reagent in which the first substance (the substance containing the 3-NPH at the concentration of 200 mM) and the second substance (the substance containing the EDC at the concentration of 200 mM and the pyridine at the concentration of 9%) are in the same amount in the derivatization.

Further, as described as step S114 in Fig. 5, control unit 401 can display the detected value for each of the 23 types of organic acids illustrated in Fig. 3 as the analysis results of the sample. In other words, in LC mass spectrometer 100, mass spectrometer 150 can derive the detected value for the each of the 23 types of organic acids, and control unit 200 can transmit the detected value for the each of the 23 types of organic acids to control unit 401. Accordingly, a doctor who visually recognizes the analysis results of the sample can determine not only the presence or the absence of the metabolic disorder but also the type of the metabolic disorder for the subject.

Further, as described as step S104 in Fig. 5, the alcohol is used as the solvent when the protein is removed from the sample. The alcohol to be used may be the methanol. The solvent when the protein is removed from the sample may be acetonitrile. By using the alcohol or the acetonitrile, the drying and solidification of the organic layer becomes unnecessary, and thus the period of time required for the pretreatment can also be shortened.

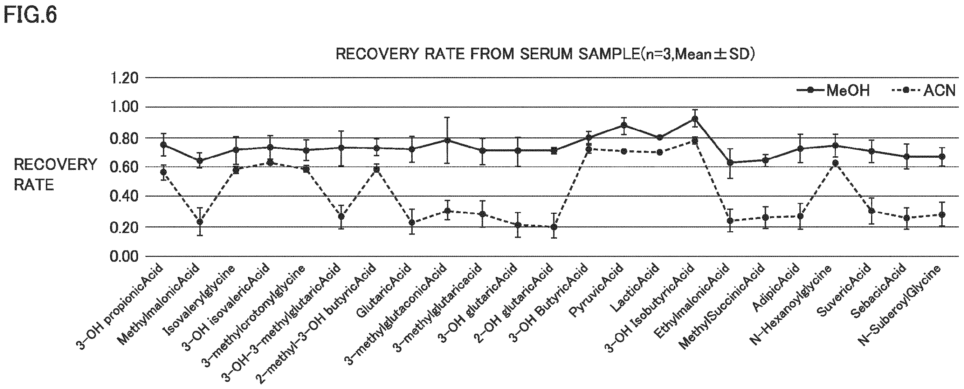

In particular, the recovery rate of the each of the 23 types of organic acids illustrated in Fig. 3 from the sample can be improved by using the methanol for removing the protein from the sample. Fig. 6 is the diagram illustrating the example of the recovery rate of the each of the 23 types of organic acids. The recovery rate represents a ratio of a value after protein removal to a value before protein removal of the concentration of the each of the organic acids in the sample. For example, a recovery rate in a case where the concentration after the protein removal is 50% of the concentration after the protein removal is "0.5". In Fig. 6, a solid line represents a recovery rate when the methanol (MeOH) is adopted as the solvent. A dashed line represents, for comparison, a recovery rate when the acetonitrile (ACN) is adopted as the solvent.

As illustrated by the dashed line, in a case where the acetonitrile is adopted as the solvent, recovery rates of some organic acids (the methylmalonic acid, the 3-hydroxy-3-methylglutaric acid, and the like) are below 0.4. On the other hand, as illustrated by the solid line, in a case where the methanol is adopted as the solvent, the recovery rates exceed 0.6 as to all the organic acids.

In analysis system 1000 of the embodiment described above, control unit 401 of pretreatment apparatus 400 integrally controls analysis system 1000. In other words, control unit 401 outputs an analysis instruction to LC mass spectrometer 100. Note that, control unit 200 of LC mass spectrometer 100 may integrally control analysis system 1000. For example, control unit 200 may output, to pretreatment apparatus 400, an instruction for performing the pretreatment (for example, the protein removal and the derivatization) on the sample and transporting the sample pretreated to LC mass spectrometer 100. In response to the instruction from control unit 200, control unit 401 may cause adding unit 402, filtering unit 404, and derivatizing unit 406 to perform the pretreatment on the sample, and may also cause transporting unit 408 to transport the sample pretreated to LC mass spectrometer 100. Further, a third apparatus different from LC mass spectrometer 100 and pretreatment apparatus 400 may integrally control analysis system 1000.

### [Variation]

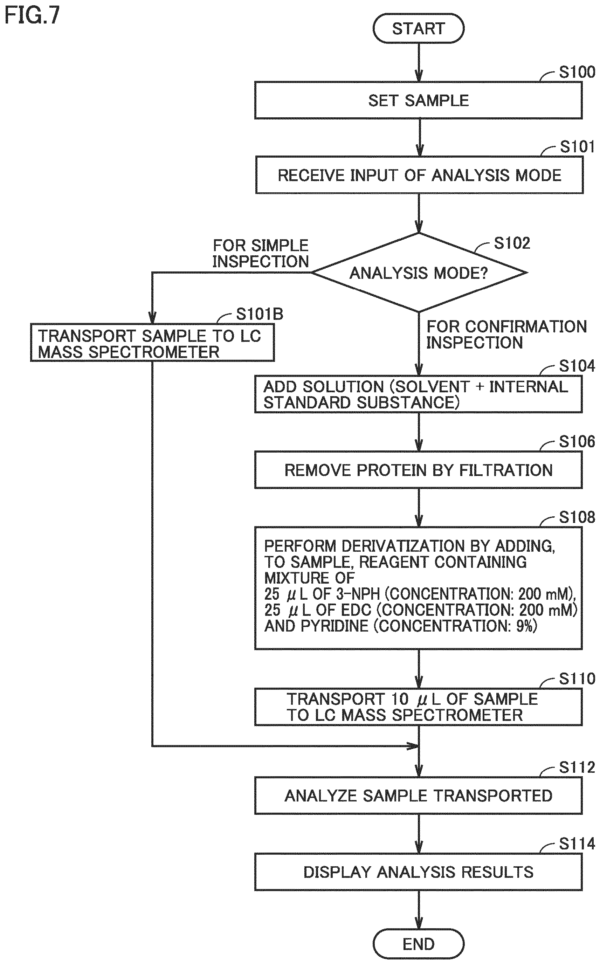

Fig. 7 is the flowchart illustrating the variation of the analysis method illustrated in Fig. 5. The analysis method illustrated in Fig. 7 further includes processes of steps S101 to S103 as compared with the analysis method illustrated in Fig. 5.

After the sample is set in step S100, control unit 401 of pretreatment apparatus 400, for example, displays that "please enter an analysis mode" on display unit 460 while receiving an input of the analysis mode in step S101. The input of the analysis mode may be an option from two types of modes. The two types of modes may be "analysis mode for simple inspection" and "analysis mode for confirmation inspection".

In step S102, control unit 401 confirms the type of the analysis mode input. If the analysis mode input is "analysis mode for simple inspection", control unit 401 advances control to step S103. If the analysis mode input is "analysis mode for confirmation inspection", control unit 401 advances the control to step S104.

In step S103, transporting unit 408 transports the sample to injector 130 of LC mass spectrometer 100. Accordingly, the sample without being derivatized is transported to LC mass spectrometer 100. Then, in step S 112, LC mass spectrometer 100 analyzes the sample without being derivatized. In step S 114, control unit 401 displays analysis results of the sample without being derivatized on display unit 460.

On the other hand, when the control proceeds to step S104, the sample derivatized is transported to LC mass spectrometer 100 as described with reference to Fig. 5 (steps S104 to S 110). Then, in step S 112, LC mass spectrometer 100 analyzes the sample derivatized. In step S 114, control unit 401 displays analysis results of the sample derivatized on display unit 460.

As described above, in the analysis method described with reference to Fig. 7, control unit 401 of pretreatment apparatus 400 receives the input of the analysis mode. Upon receiving an input designating the analysis mode for simple inspection, analysis system 1000 analyzes the sample without being derivatized and displays the analysis results. On the other hand, upon receiving an input designating the analysis mode for confirmation inspection, analysis system 1000 analyzes the sample derivatized and displays the analysis results. In other words, analysis system 1000 can output analysis results for the simple inspection or analysis results for the confirmation inspection by using LC mass spectrometer 100 according to the type of the inspection mode input.

### [Aspects]

It is understood by those skilled in the art that a plurality of the embodiments described above are specific examples of the following aspects.

(Item 1) An analysis method according to one aspect is an analysis method for a sample related to a metabolic disorder, the analysis method including: derivatizing a sample based on a metabolite of a subject by reacting with a reagent containing 3-nitrophenylhydrazine; introducing the sample derivatized into the liquid chromatograph mass spectrometry apparatus; and analyzing, by the liquid chromatograph mass spectrometry apparatus, the sample derivatized.

According to the analysis method described in Item 1, the organic acid contained in the sample is derivatized by reacting with the reagent containing the 3-nitrophenylhydrazine, and thus derivatized in a short time.

(Item 2) An analysis method according to one aspect is an analysis method for a sample related to a metabolic disorder, the analysis method including: receiving an input of an analysis type; analyzing a sample based on a metabolite of a subject according to a first flow by using a liquid chromatograph mass spectrometry apparatus in a case where the input is a first type of input; and analyzing the sample based on the metabolite of the subject according to a second flow by using the liquid chromatograph mass spectrometry apparatus in a case where the input is a second type of input, in which the first flow includes: derivatizing a reagent by reacting with a reagent containing 3-nitrophenylhydrazine; introducing the sample derivatized into the liquid chromatograph mass spectrometry apparatus; and analyzing, by the liquid chromatograph mass spectrometry apparatus, the sample derivatized, and the second flow includes: introducing the sample into the liquid chromatograph mass spectrometry apparatus without derivatizing the sample; and analyzing the sample by the liquid chromatograph mass spectrometry apparatus.

According to the analysis method described in Item 2, the liquid chromatograph mass spectrometry apparatus is used in analysis for confirmation inspection and analysis for simple inspection. Further, in the analysis for the confirmation inspection, the organic acid contained in the sample is derivatized by reacting with the reagent containing the 3-nitrophenylhydrazine, and thus derivatized in a short time.

(Item 3) The analysis method described in Item 1 or 2 further includes: adding alcohol to the sample before reaction of the sample with the reagent; and filtering the sample to which the alcohol is added.

According to the analysis method described in Item 3, drying and solidification of an organic solvent is not required in the derivatization, and thus the organic acid contained in the sample is derivatized in a short time.

(Item 4) An analysis method according to one aspect is an analysis method for a sample related to a metabolic disorder, the analysis method including: adding alcohol to a sample based on a metabolite of a subject; filtering the sample to which the alcohol is added; derivatizing, by reacting with a reagent, the sample filtered; introducing the sample derivatized into a liquid chromatograph mass spectrometry apparatus; and analyzing, by the liquid chromatograph mass spectrometry apparatus, the sample derivatized.

According to the analysis method described in Item 4, the drying and solidification of the organic solvent is not required in the derivatization, and thus the organic acid contained in the sample is derivatized in a short time.

(Item 5) In the analysis method described in any one of Items 1 to 4, derivatizing the sample includes causing the sample to react with the reagent for at least 15 minutes.

According to the analysis method described in Item 5, the derivatization of the organic acid contained in the sample is performed in a short time of 15 minutes.

(Item 6) The analysis method described in any one of Items 1 to 5 further includes outputting a predetermined concentration of a compound for the metabolic disorder in the sample as analysis results by the liquid chromatograph mass spectrometry apparatus.

According to the analysis method described in Item 6, information for determining not only presence or absence but also a type of the metabolic disorder is provided.

(Item 7) A pretreatment apparatus according to one aspect is a pretreatment apparatus performing pretreatment on a sample related to a metabolic disorder, the pretreatment apparatus including: a derivatizing unit that derivatizes a sample based on a metabolite of a subject by reacting with a reagent containing 3-nitrophenylhydrazine; and a transporting unit that introduces the sample derivatized into a liquid chromatograph mass spectrometry apparatus.

According to the pretreatment apparatus described in Item 7, the organic acid contained in the sample is derivatized by reacting with the reagent containing the 3-nitrophenylhydrazine, and thus derivatized in a short time.

(Item 8) A pretreatment apparatus according to one aspect is a pretreatment apparatus performing pretreatment on a sample related to a metabolic disorder, the pretreatment apparatus including: an input unit that receives an input of an analysis type; a derivatizing unit that derivatizes a sample by reacting with a reagent containing 3-nitrophenylhydrazine; and a transporting unit that introduces the sample into a liquid chromatograph mass spectrometry apparatus. In a case where the input is a first type of input, the derivatizing unit derivatizes a sample based on a metabolite of a subject by reacting with the reagent, and the transporting unit introduces the sample derivatized by the derivatizing unit into the liquid chromatograph mass spectrometry apparatus. In a case where the input is a second type of input, the transporting unit introduces the sample without being derivatized by the derivatizing unit into the liquid chromatograph mass spectrometry apparatus.

According to the pretreatment apparatus described in Item 8, the liquid chromatograph mass spectrometry apparatus is used in the analysis for the confirmation inspection and the analysis for the simple inspection. Further, in the analysis for the confirmation inspection, the organic acid contained in the sample is derivatized by reacting with the reagent containing the 3-nitrophenylhydrazine, and thus derivatized in a short time.

(Item 9) The pretreatment apparatus described in Item 7 or 8 further includes: an adding unit that adds alcohol to the sample before reaction of the sample with the reagent; and a filtering unit that filters the sample to which the alcohol is added.

According to the pretreatment apparatus described in Item 9, the drying and solidification of the organic solvent is not required in the derivatization, and thus the organic acid contained in the sample is derivatized in a short time.

(Item 10) A pretreatment apparatus according to one aspect is a pretreatment apparatus performing pretreatment on a sample related to a metabolic disorder, the pretreatment apparatus including: an adding unit that adds alcohol to a sample based on a metabolite of a subject; a filtering unit that filters the sample to which the alcohol is added; a derivatizing unit that derivatizes, by reacting with a reagent, the sample filtered; and a transporting unit that introduces the sample derivatized into a liquid chromatograph mass spectrometry apparatus.

According to the pretreatment apparatus described in Item 10, the drying and solidification of the organic solvent is not required in the derivatization, and thus the organic acid contained in the sample is derivatized in a short time.

(Item 11) In the pretreatment apparatus described in any one of Items 7 to 10, the derivatizing unit causes the sample to react with the reagent for at least 15 minutes.

According to the pretreatment apparatus described in Item 11, the derivatization of the organic acid contained in the sample is performed in a short time of 15 minutes.

(Item 12) The pretreatment apparatus described in any one of Items 7 to 11 further includes a display unit that displays a predetermined concentration of a compound for the metabolic disorder in the sample as analysis results of the sample acquired in the liquid chromatograph mass spectrometry apparatus.

According to the pretreatment apparatus described in Item 12, the information for determining not only the presence or the absence but also the type of the metabolic disorder is provided.

It should be considered that the embodiments disclosed herein are illustrative in all respects and not restrictive. The scope of the present disclosure is indicated by claims rather than the above description of the embodiments, and it is intended that meanings equivalent to the claims and all modifications within the scope are included. Further, it is intended that each technique in the embodiments can be implemented alone or in combination with other techniques in the embodiments whenever possible as necessary.

### REFERENCE SIGNS LIST

100: liquid chromatograph mass spectrometer (LC mass spectrometer), 110: solvent container, 120: liquid feeding pump, 130: injector, 140: column oven, 145: column, 150: mass spectrometer, 200, 401: control units, 220, 420: memory, 250, 450: input units, 260, 460: display units, 300: data processing unit, 400: pretreatment apparatus, 402: adding unit, 404: filtering unit, 406: derivatizing unit, 408: transporting unit, 1000: analysis system

## Claims

1. An analysis method for a sample related to a metabolic disorder, the analysis method comprising:
derivatizing a sample, which is based on a metabolite of a subject, by reacting with a reagent containing 3-nitrophenylhydrazine;
introducing the derivatized sample into a liquid chromatograph mass spectrometry apparatus; and
analyzing, by the liquid chromatograph mass spectrometry apparatus, the derivatized sample.

2. An analysis method for a sample related to a metabolic disorder, the analysis method comprising:
receiving an input of an analysis type;
analyzing a sample, which is based on a metabolite of a subject, according to a first flow by using a liquid chromatograph mass spectrometry apparatus in a case where the input is a first type of input; and
analyzing the sample, which is based on the metabolite of the subject, according to a second flow by using the liquid chromatograph mass spectrometry apparatus in a case where the input is a second type of input, wherein
the first flow includes:
derivatizing a reagent by reacting with a reagent containing 3-nitrophenylhydrazine;
introducing the derivatized sample into the liquid chromatograph mass spectrometry apparatus; and
analyzing, by the liquid chromatograph mass spectrometry apparatus, the derivatized sample, and
the second flow includes:
introducing the sample without derivatization into the liquid chromatograph mass spectrometry apparatus; and
analyzing the sample without derivatization by the liquid chromatograph mass spectrometry apparatus.

3. The analysis method according to claim 1 or 2, further comprising:
adding alcohol to the sample before reaction of the sample with the reagent; and
filtering the alcohol-added sample.

4. An analysis method for a sample related to a metabolic disorder, the analysis method comprising:
adding alcohol to a sample based on a metabolite of a subject;
filtering the alcohol-added sample;
derivatizing, by reacting with a reagent, the filtered sample;
introducing the derivatized sample into a liquid chromatograph mass spectrometry apparatus; and
analyzing, by the liquid chromatograph mass spectrometry apparatus, the derivatized sample.

5. The analysis method according to any one of claims 1, 2, and 4, wherein derivatizing the sample includes causing the sample to react with the reagent for at least 15 minutes.

6. The analysis method according to any one of claims 1, 2, and 4, further comprising outputting a predetermined concentration of a compound for the metabolic disorder in the sample as analysis results by the liquid chromatograph mass spectrometry apparatus.

7. A pretreatment apparatus performing pretreatment on a sample related to a metabolic disorder, the pretreatment apparatus comprising:
a derivatizing unit that derivatizes a sample, which is based on a metabolite of a subject, by reacting with a reagent containing 3-nitrophenylhydrazine; and
a transporting unit that introduces the derivatized sample into a liquid chromatograph mass spectrometry apparatus.

8. A pretreatment apparatus performing pretreatment on a sample related to a metabolic disorder, the pretreatment apparatus comprising:
an input unit that receives an input of an analysis type;
a derivatizing unit that derivatizes a sample by reacting with a reagent containing 3-nitrophenylhydrazine; and
a transporting unit that introduces the sample into a liquid chromatograph mass spectrometry apparatus, wherein
in a case where the input is a first type of input,
the derivatizing unit derivatizes a sample, which is based on a metabolite of a subject, by reacting with the reagent, and
the transporting unit introduces the derivatized sample by the derivatizing unit into the liquid chromatograph mass spectrometry apparatus, and
in a case where the input is a second type of input,
the transporting unit introduces the sample without derivatization by the derivatizing unit into the liquid chromatograph mass spectrometry apparatus.

9. The pretreatment apparatus according to claim 7 or 8, further comprising:
an adding unit that adds alcohol to the sample before reaction of the sample with the reagent; and
a filtering unit that filters the alcohol-added sample.

10. A pretreatment apparatus performing pretreatment on a sample related to a metabolic disorder, the pretreatment apparatus comprising:
an adding unit that adds alcohol to a sample based on a metabolite of a subject;
a filtering unit that filters the alcohol-added sample;
a derivatizing unit that derivatizes, by reacting with a reagent, the filtered sample; and
a transporting unit that introduces the derivatized sample into a liquid chromatograph mass spectrometry apparatus.

11. The pretreatment apparatus according to any one of claims 7, 8, and 10, wherein the derivatizing unit causes the sample to react with the reagent for at least 15 minutes.

12. The pretreatment apparatus according to any one of claims 7, 8, and 10, further comprising a display unit that displays a predetermined concentration of a compound for the metabolic disorder in the sample as analysis results of the sample acquired in the liquid chromatograph mass spectrometry apparatus.
